**Europäisches Patentamt**

**European Patent Office**

**Office européen des brevets**

(19)

(11) Veröffentlichungsnummer : **0 089 564**
**B1**

(12) **EUROPÄISCHE PATENTSCHRIFT**

(45) Veröffentlichungstag der Patentschrift :
30.10.85

(21) Anmeldenummer : 83102382.5

(22) Anmeldetag : 11.03.83

(51) Int. Cl.⁴ : **C 07 C 51/15, C 07 C 65/05,
C 07 C 65/11**

(54) Verfahren zur Herstellung von aromatischen Hydroxycarbonsäuren.

(30) Priorität : 23.03.82 DE 3210597

(43) Veröffentlichungstag der Anmeldung :
28.09.83 Patentblatt 83/39

(45) Bekanntmachung des Hinweises auf die Patenterteilung : 30.10.85 Patentblatt 85/44

(84) Benannte Vertragsstaaten :
CH DE FR GB LI

(56) Entgegenhaltungen :
DE-A- 1 643 544
DE-A- 2 926 694
DE-B- 1 493 881
DE-C-   960 206

(73) Patentinhaber : BAYER AG
Konzernverwaltung RP Patentabteilung
D-5090 Leverkusen 1 Bayerwerk (DE)

(72) Erfinder : Stopp, Gerhard, Dr.
Walter-Flex-Strasse 21
D-5090 Leverkusen (DE)
Erfinder : Karkossa, Horst
Immigrather Strasse 57
D-5653 Leichlingen (DE)
Erfinder : Trescher, Viktor, Dr.
Voelklinger Strasse 7
D-5090 Leverkusen (DE)

## Beschreibung

Die Erfindung betrifft ein Verfahren zur Herstellung von aromatischen Hydroxycarbonsäuren durch Umsetzung in fester Phase von Alkalisalzen aromatischer Hydroxyverbindungen mit Kohlendioxid.

Die Umsetzung von Alkalisalzen aromatischer Hydroxyverbindungen in fester Form mit Hilfe von Kohlendioxid zu den Alkalisalzen der entsprechenden aromatischen Hydroxycarbonsäuren ist bekannt. So wird z. B. in der DE-PS-960 206 ein Verfahren zur Umwandlung von feinkörnigen Alkalisalzen aromatischer Monohydroxyverbindungen in die Alkalisalze der entsprechenden Hydroxycarbonsäuren mit Hilfe von Kohlendioxid beansprucht, das dadurch gekennzeichnet ist, daß die Ausgangssalze in Form von Partikeln mit einem Durchmesser von 20 bis 200 μm eingesetzt und in einem flüssigkeitsähnlichen Zustand, einer sogenannten Wirbelschicht, mit Kohlendioxid begast werden.

Weiterhin ist aus der DE- AS-1 493 881 bekannt, p-Hydroxybenzoesäure durch Reaktion von Kaliumphenolat bei einer Temperatur von 190 bis 210 °C und einem Druck bis zu 6 Atü unter Verwendung eines auf Reaktionstemperatur gebrachten Kreislaufs, bestehend aus Kohlendioxid und Inertgas, herzustellen, wobei laufend für die Abführung der Reaktionswärme und des entstandenen Phenols Sorge getragen und verbrauchtes Kohlendioxid durch Frischgas ersetzt wird und wobei man das Kaliumphenolat in Form von wasserfreiem Granulat oder Preßlingen mit bis zu 30 mm Durchmesser oder Kantenlänge einsetzt. Die Reaktion erfolgt dabei in einem Festbettreaktor, der mit dem Kaliumphenolat beschickt ist und bei dem das Gas von unten nach oben geführt wird.

In der britischen Patentschrift 1 205 447 wird außerdem ein Verfahren beschrieben zur Herstellung von Alkalisalzen aromatischer Hydroxycarbonsäuren durch Umsetzung bei höherer Temperatur von Kohlendioxid mit pulverförmigen Alkalimetallphenolaten, die einen Durchmesser von weniger als 70 μm haben.

Aus der DE-OS-2 926 694 (US-PS-4 171 453) ist außerdem bekannt, durch Carbonisieren eines trockenen Alkalimetallphenolats in fester Phase mit Kohlendioxid unter Druck das Alkalimetallcarboxylat des entsprechenden Phenols herzustellen. Dieses Verfahren wird so durchgeführt, daß in der ersten Stufe Kohlendioxid mit feinverteiltem, festem Alkalimetallphenolat bei einer Temperatur unter 135 °C umgesetzt wird, bis mindestens 25 % der stöchiometrischen Menge an Kohlendioxid von dem Phenolat absorbiert sind und in der zweiten Stufe durch Erhöhung der Temperatur auf über 135 °C die Carbonisierung des Phenolats weitergeführt wird.

Nachteilig bei den aus dem Stand der Technik bekannten Verfahren sind die z. T. unbefriedigenden Ausbeuten an aromatischen Hydroxycarbonsäuren (DE-AS-1 493 881 und GB-PS-1 205 447), die Probleme, die sich beim Einsatz von pulverförmigem Ausgangsmaterial ergeben, mit der aus Sicherheitsgründen mit einem hohen technischen Aufwand verbundenen Handhabung des pulverförmigen Materials (DE-PS-960 206 und GB-PS-1 205 447) sowie der mit einem Kohlendioxid-Verlust und Abluftproblemen einhergehende Einsatz von Inertgas zur Kühlung und zur Abführung von während der Reaktion gebildetem Phenol (DE-AS-1 493 881). Weiterhin ist bei dem Verfahren der DE-PS-960 206 nachteilig, daß unter den dort beschriebenen Reaktionsbedingungen die freie aromatische Hydroxyverbindung gebildet wird, die auch durch absorbierende Zusätze, wie Kaolin, nicht gebunden wird und auf diese Weise zu Verbackungen und Verklebungen der Reaktionsprodukte führt.

Verfahren zur Herstellung von aromatischen Hydroxycarbonsäuren durch Umsetzung in fester Phase von Alkalisalzen aromatischer Hydroxyverbindungen mit Kohlendioxid, das dadurch gekennzeichnet ist, daß man das Alkalisalz der aromatischen Hydroxyverbindung in Form von Granulat in einem Wirbelbett in Gegenwart von Kohlendioxid, gegebenenfalls bei erhöhtem Druck und gegebenenfalls in Gegenwart von dampfförmiger aromatischer Hydroxyverbindung, unter ständiger Abführung von entstehender aromatischer Hydroxyverbindung mit überschüssigem Kohlendioxid bei Temperaturen von 140 bis 300 °C umsetzt, bis sich mindestens 40 % der Hydroxyverbindung in das Alkalisalz der entsprechenden Hydroxycarbonsäure umgewandelt haben und daß man gegebenenfalls in einer nachfolgenden Stufe das erhaltene Alkalisalz der Hydroxycarbonsäure mit der gebildeten aromatischen Hydroxyverbindung in Gegenwart von Kohlendioxid bei Temperaturen von 150 bis 300 °C und bei erhöhtem Druck weiter umsetzt und danach das erhaltene Reaktionsprodukt in üblicher Weise in die freie Säure überführt.

Als Alkalisalze aromatischer Hydroxyverbindungen können in das erfindungsgemäße Verfahren die Natrium- und/oder Kaliumsalze des Phenols, der Kresole, der Naphthole, des 2-Hydroxycarbazols oder des 3-Hydroxydiphenylenoxids eingesetzt werden. Die aromatischen Hydroxyverbindungen können durch niederes Alkyl, wie Methyl, Ethyl oder tert.-Butyl, sowie durch Halogene, wie Fluor, Chlor, Brom, bevorzugt Chlor, einfach oder mehrfach substituiert sein. Bevorzugt werden in das erfindungsgemäße Verfahren Natriumphenolat, die Natriumsalze der Kresole sowie die Natriumsalze der Chlorphenole, besonders bevorzugt Natriumphenolat, eingesetzt.

Die Alkalisalze der aromatischen Hydroxyverbindungen werden in das erfindungsgemäße Verfahren in Form von wasserfreiem Granulat mit einem Durchmesser von etwa 0,2 bis 5 mm, bevorzugt 0,3 bis 4 mm, und einer inneren Oberfläche von 1 bis 6 m²/g, bevorzugt 2 bis 4 m²/g (Oberfläche bestimmt nach BET-Methode/DIN 66 132) eingesetzt.

Das erfindungsgemäß einzusetzende Granulat kann hergestellt werden nach einem eigenen, älteren Verfahren, das in der noch nicht veröffentlichten deutschen Patentanmeldung P 3 206 236.2 dargelegt ist.

Nach dem erfindungsgemäßen Verfahren setzt man die granulierten Salze der aromatischen Hydroxyverbindungen in einem Wirbelbett mit dem Kohlendioxid, das gegebenenfalls die freie aromatische Hydroxyverbindung in Dampfform enthalten kann, um. Das Kohlendioxid wird dabei im Kreis gefahren, wobei die Reaktionswärme und die während der Reaktion entstandene aromatische Hydroxyverbindung mit überschüssigem Kohlendioxid abgeführt werden. Aus dem im Kreis geführten Kohlendioxid kann dabei die aromatische Hydroxyverbindung, durch z. B. Kühlung, abgetrennt werden. Die Menge an Kohlendioxid die während der Umsetzung verbraucht worden ist, wird dem im Kreis geführten Kohlendioxid jeweils wieder zugesetzt.

Man führt die Umsetzung in dem Wirbelbett bevorzugt bei Temperaturen von 150 bis 250 °C durch. Die Drucke betragen dabei etwa 1 bis 50 $bar_{abs}$, bevorzugt 1 bis 6 $bar_{abs}$.

Nachdem sich etwa 40 %, bevorzugt 40 bis 50 %, der aromatischen Hydroxyverbindung mit dem Kohlendioxid zu dem Alkalisalz der entsprechenden Hydroxycarbonsäure umgesetzt haben, kann man die Reaktion beenden und das danach erhaltene Reaktionsprodukt in üblicher Weise in die freie Säure überführen.

Es hat sich bei einigen Salzen der aromatischen Hydroxyverbindungen, wie dem Natriumphenolat und dem Natriumkresolat, als vorteilhaft erwiesen, daß man in einer nachfolgenden Stufe das erhaltene Alkalisalz der entsprechenden Hydroxycarbonsäure mit der gebildeten aromatischen Hydroxyverbindung in Gegenwart von Kohlendioxid bei Temperaturen von 150 bis 300 °C, bevorzugt bei Temperaturen von 160 bis 240 °C, und bei Drucken von 2 bis 50 $bar_{abs}$, bevorzugt bei 4 bis 10 $bar_{abs}$, weiter umsetzt, d. h. bis kein Kohlendioxid mehr aufgenommen wird. Dabei kann in der nachfolgenden Stufe neben der bereits gebildeten aromatischen Hydroxyverbindung noch eine zusätzliche Menge der entsprechenden aromatischen Hydroxyverbindung (etwa 5 bis 15 Mol-%) zugegeben werden.

Diese nachfolgende Stufe kann, statt in einem Wirbelbett, auch in einer anderen Apparatur, wie einem Rührwerksbehälter oder einer Schneckenmaschine, durchgeführt werden.

Zur Aufarbeitung des Reaktionsgemisches wird gegebenenfalls überschüssige aromatische Hydroxyverbindung durch Vakuumdestillation aus dem Reaktionsgemisch entfernt, das Reaktionsgemisch anschließend mit Wasser versetzt, neutralisiert und gegebenenfalls durch Zugabe von Zusatzstoffen, wie Aktiv-Kohle, geklärt und mit Mineralsäuren, wie Schwefelsäure oder Salzsäure, gefällt, wobei man die freie aromatische Hydroxycarbonsäure erhält.

Das erfindungsgemäße Verfahren kann wie folgt durchgeführt werden (gezeigt am Beispiel der Herstellung von Salicylsäure) : Das trockene, granulierte Natriumphenolat wird dem Wirbelbettreaktor kontinuierlich zugeführt und mit dem von unten nach oben strömenden, phenolhaltigen Kohlendioxid, das im Kreis geführt wird, bei etwa 140 bis 170 °C und einem Druck von etwa 5 bis 10 $bar_{abs}$ umgesetzt. Das als Kreisgas dienende Kohlendioxid, das zur Abführung von entstehendem Phenol und zur Abführung der Reaktionswärme dient, wird dabei jeweils um den Anteil an Kohlendioxid ergänzt, der bei der Umsetzung mit der Hydroxyverbindung verbraucht worden ist. Nachdem das Natriumphenolat ca. 50 % der theoretisch möglichen Menge an Kohlendioxid aufgenommen hat, wird das entstandene Natriumsalz der Salicylsäure in einen Rührwerksbehälter überführt und dort in Gegenwart von Kohlendioxid mit dem gebildeten Phenol unter weiterer Zugabe von ca. 10 Mol-% Phenol bei Temperaturen von etwa 160 bis 210 °C unter einem Druck von etwa 5 bis 10 $bar_{abs}$ weiterumgesetzt.

In einer sich anschließenden Vakuumdestillation (ca. 0,01 $bar_{abs}$) werden Restmengen an Phenol aus dem Reaktionsgemisch entfernt und anschließend das Reaktionsgemisch in Wasser gelöst und mit 50 %iger Schwefelsäure versetzt, wobei die freie Salicylsäure erhalten wird.

Die nach dem erfindungsgemäßen Verfahren erzielten Ausbeuten an aromatischen Hydroxycarbonsäuren betragen je nach eingesetztem Alkalisalz der aromatischen Hydroxyverbindung etwa 50 bis 96 % der Theorie, bezogen auf die Ausgangsverbindung.

Das erfindungsgemäße Verfahren kann sowohl kontinuierlich als auch diskontinuierlich durchgeführt werden.

Die Vorteile des erfindungsgemäßen Verfahrens sind insbesondere zu sehen in der Vermeidung einer flüssigen phenolischen Phase, wodurch Verklebungen und Verbackungen der Reaktionsprodukte verhindert werden. Damit erreicht man wesentlich verkürzte Reaktionszeiten bei geringem Energieverbrauch. Durch die Möglichkeit einer genauen Temperaturführung kann die Bildung von Nebenprodukten minimiert werden. Außerdem können bei dem erfindungsgemäßen Verfahren Verfärbungen der Reaktionsprodukte durch Luftoxidation vermieden werden. Schließlich erhält man nach dem erfindungsgemäßen Verfahren die aromatischen Hydroxycarbonsäuren in guten Ausbeuten mit einer hohen Selektivität.

Bei dem erfindungsgemäßen Verfahren überrascht besonders, daß das im Wirbelbett behandelte Granulat auch nach der Umsetzung mit dem Kohlendioxid in fester Form erhalten bleibt und so ohne Probleme dem Wirbelbettreaktor entnommen werden kann. Ebenso bleibt die Granulatform bei der bei höheren Temperaturen durchgeführten Vervollständigung der Reaktion erhalten, was besonders günstig für die technische Durchführung des erfindungsgemäßen Verfahren sowie für die Weiterverarbeitung der Reaktionsprodukte ist. Weiterhin ist überraschend, daß trotz der Grobkörnigkeit der eingesetzten Alkalisalze aromatischer Hydroxyverbindungen keine Beeinträchtigung der Umsetzungsgeschwindigkeit und der Ausbeute eintritt, obwohl in Ind. J. Technol. Vol. 11, S. 187 (1973) darauf hingewiesen wird, daß mit zunehmender Teilchengröße von Natriumphenolat die Reaktionsgeschwindigkeit der Carboxylierung abnimmt.

Die nach dem erfindungsgemäßen Verfahren hergestellten aromatischen Hydroxycarbonsäuren sind wertvolle Zwischenprodukte für die Herstellung von Farbstoffen, Arzneimitteln, Pflanzenschutzmitteln, Gerbstoffen und Kosmetika (Ullmanns Encyl. d. techn. Chemie, Bd. 13, 4. Aufl., S. 163-168).

Die nachfolgenden Beispiele sollen das erfindungsgemäße Verfahren verdeutlichen, ohne es jedoch auf diese Beispiele einzuschränken.

Beispiel 1

Ein kontinuierlicher Strom von 8 Teilen/h trockenem, granulierten Kaliumphenolat (Hauptanteil : 0,6 bis 1,2 mm Durchmesser) wird über eine geeignete Eintragsvorrichtung in ein Wirbelbett eingetragen. Als Fluidisiergas dient Kohlendioxid, welches gleichzeitig das Reaktionsgas darstellt. Das Reaktionsgas wird im Kreis gefahren. Der Druck beträgt 1,5 bar$_{abs}$. Beim Eintritt des Kaliumphenolats in die Kohlendioxidatmosphäre wird spontan Phenol abgespalten, welches im Wirbelbett verteilt und verdampft wird. Es wird mit dem Kreisgas ausgetragen und aus diesem durch Kühlung entfernt. Das von dem Phenol weitgehend befreite Kohlendioxid wird dann dem Wirbelbett wieder zugeführt. Die Temperatur im Wirbelbett wird bei 240 °C gehalten.

Bei einer Verweilzeit von maximal 40 Minuten werden 6,5 Teile/h Dikalium-p-hydroxybenzoat erhalten. Diese werden als Granulat kontinuierlich aus dem Wirbelbett entnommen. Die Ausbeute an p-Hydroxybenzoesäure, bezogen auf das verbrauchte Phenol, beträgt über 98 %. Das Salz ist von hoher Reinheit und kann in üblicher Weise mit Mineralsäuren in die freie Säure überführt werden.

Beispiel 2

Ein kontinuierlicher Strom von 5,0 Teilen/h granuliertem, trockenen Natrium-α-naphtholat wird in ein Wirbelbett eingetragen. Als Fluidisiergas dient Kohlendioxid, welches im Kreis geführt und bei einem konstanten Druck von 5 bar$_{abs}$ gehalten wird. Das durch die Reaktion verbrauchte Kohlendioxid wird über die Druckhaltung ergänzt.

Es wird im Wirbelbett eine Temperatur von 150 °C gehalten, wobei die Reaktionswärme durch das Kreisgas abgeführt wird. Es setzen sich ca. 70 % des Naphtholats mit Kohlendioxid um. Aus dem Wirbelbett wird bei einer Verweilzeit von 5 bis 20 Minuten ein kontinuierlicher Granulatstrom entnommen und einem nachfolgenden Wirbelbett zugeführt. In diesem wird die Reaktion bei 170 °C unter einem Kreisgasstrom von Kohlendioxid bei einem Druck von 4,8 bar$_{abs}$ zu Ende geführt. Geringe Mengen von nicht umgesetztem Naphthol werden durch Kühlung aus dem Kreisgas zurückgewonnen.

Das Reaktionsgranulat wird kontinuierlich aus diesem zweiten Wirbelbett bei einer Verweilzeit unter 10 Minuten entnommen und über eine Kühlstrecke geleitet. Man erhält 6,0 Teile des Natriumsalzes der 1-Hydroxynaphthalincarbonsäure-2, das entspricht einer Ausbeute von 95 %.

Das Reaktionsprodukt besitzt eine hohe Reinheit und kann direkt als Zwischenprodukt weiter verarbeitet werden oder es wird in üblicher Weise in Wasser gelöst, neutralisiert, geklärt und mit Mineralsäuren in die freie Säure überführt.

**Patentansprüche**

1. Verfahren zur Herstellung von aromatischen Hydroxycarbonsäuren durch Umsetzung in fester Phase von Alkalisalzen aromatischer Hydroxyverbindungen mit Kohlendioxid, dadurch gekennzeichnet, daß man das Alkalisalz der aromatischen Hydroxyverbindung in Form von Granulat in einem Wirbelbett in Gegenwart von Kohlendioxid, gegebenenfalls bei erhöhtem Druck und gegebenenfalls in Gegenwart von dampfförmiger aromatischer Hydroxyverbindung, unter ständiger Abführung von entstehender aromatischer Hydroxyverbindung mit überschüssigem Kohlendioxid bei Temperaturen von 140 bis 300 °C umsetzt, bis sich mindestens 40 % der Hydroxyverbindung in das Alkalisalz der entsprechenden Hydroxycarbonsäure umgewandelt haben und daß man gegebenenfalls in einer nachfolgenden Stufe das erhaltene Alkalisalz der Hydroxycarbonsäure mit der gebildeten aromatischen Hydroxyverbindung in Gegenwart von Kohlendioxid bei Temperaturen von 150 bis 300 °C und bei erhöhtem Druck weiter umsetzt und danach das erhaltene Reaktionsprodukt in üblicher Weise in die freie Säure überführt.

2. Verfahren nach Anspruch 1, dadurch gekennzeichnet, daß man als Alkalisalze aromatischer Hydroxyverbindungen die Natrium- und-/oder Kaliumsalze des Phenols, der Kresole, der Naphthole, des 2-Hydroxycarbazols oder des 3-Hydroxydiphenylenoxids einsetzt.

3. Verfahren nach Ansprüchen 1 und 2, dadurch gekennzeichnet, daß man die Alkalisalze aromatischer Hydroxyverbindungen in Form von Granulat mit einem Durchmesser von 0,2 bis 5 mm und einer inneren Oberfläche von 1 bis 6 m$^2$/g einsetzt.

4. Verfahren nach Ansprüchen 1 und 3, dadurch gekennzeichnet, daß man die Alkalisalze aromatischer Hydroxyverbindungen in Form von Granulat mit einem Durchmesser von 0,3 bis 4 mm und einer inneren Oberfläche von 2 bis 4 m²/g einsetzt.

5. Verfahren nach Ansprüchen 1 bis 4, dadurch gekennzeichnet, daß man die Umsetzung gegebenenfalls bei Drucken von 1 bis 50 bar$_{abs}$ durchführt.

6. Verfahren nach Ansprüchen 1 bis 5, dadurch gekennzeichnet, daß man die Umsetzung bei Temperaturen von 150 bis 250 °C durchführt.

7. Verfahren nach Ansprüchen 1 bis 6, dadurch gekennzeichnet, daß man das Alkalisalz der aromatischen Hydroxyverbindung mit Kohlendioxid umsetzt, bis sich mindestens 40 bis 50 % der aromatischen Hydroxyverbindung zu dem Alkalisalz der entsprechenden Hydroxycarbonsäure umgewandelt haben.

8. Verfahren nach Ansprüchen 1 bis 7, dadurch gekennzeichnet, daß man in der nachfolgenden Stufe bei einem Druck von 2 bis 50 bar$_{abs}$ arbeitet.

## Claims

1. Process for the preparation of aromatic hydroxy-carboxylic acids by reacting alkali metal salts of aromatic hydroxy compounds in the solid phase with carbon dioxide, characterised in that the alkali metal salt of the aromatic hydroxy compound is reacted at temperatures of 140 to 300 °C, in the form of granules, in a fluidised bed in the presence of carbon dioxide, if appropriate under elevated pressure and, if appropriate, in the presence of the aromatic hydroxy compound in the form of vapour, with continuous removal, by means of excess carbon dioxide, of the aromatic hydroxy compound which is formed, until at least 40 % of the hydroxy compound have been converted into the alkali metal salt of the corresponding hydroxycarboxylic acid, and, if appropriate, the resulting alkali metal salt of the hydroxycarboxylic acid is reacted further in a subsequent stage at temperatures of 150 to 300 °C and under elevated pressure, in the presence of carbon dioxide, with the aromatic hydroxy compound formed, and the resulting reaction product is then converted in a customary manner into the free acid.

2. Process according to Claim 1, characterised in that the sodium and/or potassium salts of phenol, cresols, naphthols, 2-hydroxycarbazole or 3-hydroxydiphenylene oxide are employed as the alkali metal salts of aromatic hydroxy compounds.

3. Process according to Claims 1 and 2, characterised in that the alkali metal salts of aromatic hydroxy compounds are employed in the form of granules having a diameter of 0.2 to 5 mm and an internal surface area of 1 to 6 m²/g.

4. Process according to Claims 1 and 3, characterised in that the alkali metal salts of aromatic hydroxy compounds are employed in the form of granules having a diameter of 0.3 to 4 mm and an internal surface area of 2 to 4 m²/g.

5. Process according to Claims 1 to 4, characterised in that the reaction is carried out, if appropriate, under pressures of 1 to 50 bar, absolute.

6. Process according to Claims 1 to 5, characterised in that the reaction is carried out at temperatures of 150 to 250 °C.

7. Process according to Claims 1 to 6, characterised in that the alkali metal salts of the aromatic hydroxy compound is reacted with carbon dioxide until at least 40 to 50 % of the aromatic hydroxy compound have been converted into the alkali metal salt of the corresponding hydroxycarboxylic acid.

8. Process according to Claims 1 to 7, characterised in that the reaction in the subsequent stage is carried out under a pressure of 2 to 50 bar, absolute.

## Revendications

1. Procédé de préparation d'acides hydroxy-carboxyliques aromatiques par réaction en phase solide de sels alcalins de composés aromatiques hydroxylés avec l'anhydride carbonique, caractérisé en ce que l'on fait réagir le sel alcalin du composé aromatique hydroxylé sous forme de granulés dans un lit tourbillonnaire en présence d'anhydride carbonique, éventuellement sous pression et éventuellement en présence du composé aromatique hydroxylé à l'état de vapeur, avec évacuation continue du composé aromatique hydroxylé formé, avec un excès d'anhydride carbonique à des températures de 140 à 300 °C, jusqu'à ce qu'on ait converti au moins 40 % du composé hydroxylé en le sel alcalin de l'acide hydroxycarboxylique correspondant, et en ce que, éventuellement dans un stade opératoire subséquent, on fait à nouveau réagir le sel alcalin d'acide hydroxycarboxylique obtenu avec le composé aromatique hydroxylé formé en présence d'anhydride carbonique à des températures de 150 à 300 °C et sous pression, après quoi on convertit le produit de réaction obtenu en l'acide libre de la manière habituelle.

2. Procédé selon la revendication 1, caractérisé en ce que l'on utilise en tant que sels alcalins de composés aromatiques hydroxylés les sels de sodium et/ou de potassium du phénol, des crésols, des naphtols, du 2-hydroxycarbazole ou de l'oxyde de 3-hydroxydiphénylène.

3. Procédé selon les revendications 1 et 2, caractérisé en ce que l'on met en œuvre les sels alcalins de composés aromatiques hydroxylés sous forme de granulés de diamètre 0,2 à 5 mm avec une surface interne de 1 à 6 m²/g.

4. Procédé selon les revendications 1 et 3, caractérisé en ce que l'on met en œuvre les sels alcalins de composés aromatiques hydroxylés à l'état de granulés de diamètre 0,3 à 4 mm avec une surface interne de 2 à 4 m²/g.

5. Procédé selon les revendications 1 à 4, caractérisé en ce que l'on effectue la réaction éventuellement à des pressions absolues de 1 à 50 bars.

6. Procédé selon les revendications 1 à 5, caractérisé en ce que l'on effectue la réaction à des températures de 150 à 250 °C.

7. Procédé selon les revendications 1 à 6, caractérisé en ce que l'on fait réagir le sel alcalin du composé aromatique hydroxylé avec l'anhydride carbonique jusqu'à ce qu'on ait converti au moins 40 à 50 % du composé aromatique hydroxylé en le sel alcalin de l'acide hydroxycarboxylique correspondant.

8. Procédé selon les revendications 1 à 7, caractérisé en ce que, dans le stade opératoire subséquent, on opère à une pression absolue de 2 à 50 bars.